**Europäisches Patentamt**

**European Patent Office**

**Office européen des brevets**

(19)

(11) Veröffentlichungsnummer : **0 122 372**
**B1**

## (12) EUROPÄISCHE PATENTSCHRIFT

(45) Veröffentlichungstag der Patentschrift :
09.09.87

(21) Anmeldenummer : **84101094.5**

(22) Anmeldetag : **03.02.84**

(51) Int. Cl.⁴ : **A 61 F   2/66**

(54) **Künstlicher Fuss.**

(30) Priorität : **18.03.83 DE 3309777**

(43) Veröffentlichungstag der Anmeldung :
**24.10.84 Patentblatt 84/43**

(45) Bekanntmachung des Hinweises auf die Patenterteilung : **09.09.87 Patentblatt 87/37**

(84) Benannte Vertragsstaaten :
**FR GB IT SE**

(56) Entgegenhaltungen :
**DE-C-   456 941**
**DE-U- 1 879 146**
**GB-A- 1 371 996**
**US-A- 3 098 239**
**US-A- 3 833 941**
**US-A- 4 328 594**
**Soviet Inventions Illustrated week C 27, 13 August 1980**

(73) Patentinhaber : **Otto Bock Orthopädische Industrie Besitz- und Verwaltungs-Kommandit-gesellschaft Industriestrasse**
**D-3408 Duderstadt 1 (DE)**

(72) Erfinder : **Haupt, Werner**
**Friedensstrasse 39**
**D-3408 Duderstadt 25 (DE)**

(74) Vertreter : **Lins, Edgar, Dipl.-Phys. et al**
**Patentanwälte Gramm + Lins Theodor-Heuss-Strasse 2**
**D-3300 Braunschweig (DE)**

EP 0 122 372 B1

**Beschreibung**

Die Erfindung betrifft einen künstlichen Fuß mit einem sich in den Spann- und Knöchelbereich erstreckenden Kern aus inkompressiblem Material, dessen Unterseite schräg von vorn nach hinten ansteigend verläuft, mit einer den Kern mit Ausnahme einer Beinanschlußfläche umhüllenden, die Fußform bildenden Schicht aus einem Polyurethanschaum, in die im Fersenbereich unterhalb des Kerns ein Fersenkeil eingesetzt ist, wobei unterhalb der Beinanschlußfläche eine vertikale Bohrung durch den Fuß hindurchläuft, deren Durchmesser nach unten im Bereich des Kerns stufenförmig vergrößert ist, die zur Aufnahme einer Verschraubung dient und in dem Bohrungsteil mit dem größeren Durchmesser mit dem Polyurethanschaum ausgekleidet ist.

Derartige künstliche Füße, die kein Knöchelgelenk aufweisen, sind seit Jahren bekannt. Die GB-A-1 371 996 beschreibt einen derartigen Fuß, bei dem der Kern mit Ausnahme der Beinanschlußfläche vollständig von einem hautbildenden Polyurethanschaum umgeben ist. Zur Anpassung der Laufeigenschaften kann im Fersenbereich ein Keil oder mehrere Keile mit einem Kunststoffschaum einer unterschiedlichen Dichte eingesetzt werden.

Durch die US-A-3 098 239 ist ein künstlicher Fuß mit einem ähnlichen Aufbau bekannt, bei dem unterhalb des Kerns ein weichelastischer Fersenkeil vorgesehen ist, der an seiner Unterseite von einer relativ harten Sohle begrenzt wird. Die Oberseite des Fußes ist durch einen mittelhartelastischen hydrolysefesten Kunststoff gebildet, der den Fußaufbau schützen soll.

Das wesentliche Problem bei gelenklosen Füßen besteht in der Erzeugung eines möglichst guten Abrollvorganges, der einen möglichst natürlichen Gang des Prothesenträgers erlaubt. Hierfür ist es erforderlich, daß der Fersenkeil mit einem weichelastischen Material gebildet ist, das beim Aufsetzen des Fußes ausreichend eingedrückt wird. Andererseits muß die Gesamtanordnung aber hart genug sein, um eine ausreichende Stabilität beim Gehvorgang zu gewährleisten. Dieser Zusammenhang wird in der GB-A-1 371 996 nicht näher erläutert. Gut bewährt hat sich in dieser Hinsicht der künstliche Fuß entsprechend der US-A-3 098 239, der daher bei Weiterentwicklungen keiner Veränderung unterworfen worden ist. Nachteilig an dem bekannten Aufbau ist jedoch, daß der Fersenkeil und die Sohle aus saugfähigen und porösen Schaumstoffen gebildet sind, die durch Agenzien angegriffen werden und sich vollsaugen, wenn sie mit Feuchtigkeit in Berührung kommen. Der Träger des künstlichen Fußes war daher genötigt, sehr sorgsam darauf zu achten, daß der künstliche Fuß derartigen Umgebungen möglichst nicht ausgesetzt wurde. Hieraus ergab sich eine sehr starke Beeinträchtigung der Bewegungsfreiheit des Prothesenträgers.

Der Erfindung liegt daher die Aufgabe zugrunde, einen künstlichen Fuß der eingangs erwähnten Art zu erstellen, der weder durch übliche Agenzien noch durch Feuchtigkeit angegriffen bzw. in seinem Tragekomfort verschlechtert wird und der die bisher erzielbaren guten Laufeigenschaften eines gelenklosen künstlichen Fußes aufweist.

Diese Aufgabe wird erfindungsgemäß dadurch gelöst, daß der Fersenkeil aus einem Vulkollanschaum mit einer spezifischen Masse von etwa 3 g/cm³ gebildet, an die schräg verlaufende Unterseite des Kerns angesetzt und seitlich sowie an seiner Unterseite von einer etwa 2 mm starken Wand des Polyurethanschaums mit einer spezifischen Masse von etwa 6 g/cm³ umgeben ist.

Bei dem erfindungsgemäßen künstlichen Fuß füllt der Fersenkeil im Fersenbereich den Raum unterhalb der schräg verlaufenden Unterseite aus und wird seitlich sowie an seiner Unterseite nur von einer etwa 2 mm starken Wand des Polyurethanschaums umgeben. Der Polyurethanschaum hat eine spezifische Masse von etwa 6 g/cm³ und ist hydrolysefest. Der Polyurethanschaum umgibt alle Außenseiten des Fersenkeils und bildet, abgesehen von der Beinanschlußfläche, eine nahtlos geschlossene Umhüllung. Der Vulkollanschaum ist mit einer spezifischen Masse von etwa 3 g/cm³ weicher als bisherige Fersenkeile ausgebildet. Diese bei dem Fuß gemäß der US-A-3 098 239 zu einem schwimmenden, instabilen Gang führende und daher nicht mehr vertretbare Weichheit wird bei dem erfindungsgemäßen Fuß dadurch kompensiert, daß der Fersenkeil nach außen hin von dem mittelhartelastischen Polyurethanschaum umgeben ist, der die Stabilität beim Abrollvorgang trotz des sehr weich ausgebildeten Fersenkeils sichert. Um die Weichheit des Fersenkeils wirksam werden zu lassen, wird die Wandstärke von etwa 2 mm für den Polyurethanschaum im Bereich des Fersenkeils nicht überschritten.

Während in der Praxis unter dem Gesichtspunkt der Abrollfunktion des Fußes immer davon ausgegangen worden war, daß der Fersenkeil mit einer definierten Weichheit in den Fußaufbau eingesetzt wird, wobei er seitlich frei bleiben muß, um aufgrund einer Ausdehnungsmöglichkeit seine definierte Weichheit wirksam werden zu lassen, wird bei dem erfindungsgemäßen künstlichen Fuß die Weichheit des Fersenkeils durch die Härte der den Fersenkeil umgebenden Wand aus Polyurethanschaum beeinträchtigt, so daß eine resultierende Weichheit entsteht, wenn die Wand etwa 2 mm dünn ausgeführt wird. Hieraus ergibt sich der Vorteil, daß die bisherigen Laufeigenschaften des künstlichen Fußes, beispielsweise gemäß der US-A-3 098 239, unter gleichzeitiger Bildung einer den Fuß mit Ausnahme der Beinanschlußfläche umhüllenden, hydrolysefesten Schicht ermöglicht werden.

Gegenüber dem Fuß gemäß der US-A-3 098 239 zeichnet sich der erfindungsgemäße Fuß durch einen zweiteiligen Aufbau aus Fersenkeil und umhüllender Polyurethanschaumschicht aus. Der hydrolysefeste Polyurethanschaum ist etwas weicher ausgebildet als der bisher für die Oberseite verwendete Polyurethanschaum. In diesem Fall sind die Abrolleigenschaften auch im vorderen Bereich vergleichbar, weil die Gesamthärte des Zehenbereichs beim erfindungsgemäßen Fuß etwa die gleich ist, wie bei dem bisherigen Aufbau aus dem härteren hydrolysefesten Polyurethanschaum und der weicheren Sohle in diesem Bereich.

Es ist vorteilhaft, wenn der durch den Durchmessersprung der Bohrung gebildete Flansch mit einer Dichtscheibe abgedeckt ist, die beim Einsetzen und Festziehen der die Verbindung mit dem Beinteil herstellenden Verschraubung für eine Abdichtung der Bohrung nach oben hin sorgt.

Zur Regulierung der Weichheit des Fersenkeils für unterschiedliche Anwendungsfälle kann es vorteilhaft sein, die Weichheit des Fersenkeils durch einen Hohlraum zu erhöhen. Auf diese Weise lassen sich Weichheitsgrade erzielen, die allein durch die Materialauswahl für den Fersenkeil nicht mehr erreichbar wären. In einer bevorzugten Ausführungsform ist der Hohlraum durch mehrere vertikale Kanäle gebildet. Diese sind vorzugsweise als Sackbohrungen ausgebildet, deren offenes Ende zum Kern zeigt und von diesem vollständig geschlossen wird. Durch diese Ausführungsform wird neben der Weichheit eine ausreichende Stabilität des Fersenkeils erzeugt, der an seiner durch die offenen Kanäle geschwächten Oberseite flächig mit dem Kern verklebt sein kann.

Für die seitliche Stabilität beim Laufen ist es zweckmäßig, wenn beidseitig der Längsachse des Fußes eine gleiche Anzahl von Kanälen vorgesehen ist.

Die Erfindung soll im folgenden anhand eines in der Zeichnung dargestellten Ausführungsbeispiels näher erläutert werden. Es zeigen :

Figur 1  eine Draufsicht auf einen erfindungsgemäßen künstlichen Fuß ;

Figur 2  einen vertikalen Schnitt durch den Fuß aus Figur 1 entlang der Linie II-II

Der künstliche Fuß weist einen Kern 1 aus einem inkompressiblen Material auf, das im dargestellten Ausführungsbeispiel Holz ist. Der Kern 1 erstreckt sich von einer den Fuß nach oben abschließenden, ebenen Beinanschlußfläche 2 in den Knöchelbereich und Spannbereich des Fußes hinein. Seine Unterseite 3 steigt zum Fersenende des Fußes schräg nach oben an. An ihr schließt sich ein Fersenkeil 4 an, der nach vorn hin spitz zuläuft und aus einem sehr weichelastischen Schaumstoff (Vulkollan mit einer spezifischen Masse von 3 g/cm³) besteht. In dem Fersenkeil sind vertikale Kanäle 5 vorgesehen, die als Sackbohrungen ausgebildet sind und deren offenes Ende zur Unterseite 3 des Kerns 1 zeigt. Der Fersenkeil 4 ist mit der Unterseite 3 des Kerns 1 flächig verbunden. An den Teil der Unterseite 3 des Kerns 1, an den sich nicht der Fersenkeil 4 anschließt, ist ein flächiges, sich nach vorn bis in den Zehenbereich erstreckendes Textilband t angelegt, das in bekannter Weise den vorderen Bereich des Fußes verstärkt und zur Steuerung des Abrollvorganges im vorderen Bereich des Fußes dient. Die gesamte Anordnung aus Kern 1, Fersenkeil 4 und Textilband 6 ist — mit Ausnahme der Beinanschlußfläche 2 — vollständig von einem mittelhartelastischen Kunststoff 7 ummantelt, der somit sowohl den Zehenbereich 8 und die Sohle 9 als auch eine Schutzwandung 10 für den Fersenkeil und eine Schutzwandung 11 für den Kern 1 bildet. Der hydrolysefeste Kunststoff 7 ist aus einem Polyurethanschaum mit einer spezifischen Masse von 6 g/cm³ gebildet, der an der Oberfläche geschlossenporig ist. Die Wandstärke der Wand 10 und des unter dem Fersenkeil 4 befindlichen Teils der Sohle 9 ist sehr dünn und beträgt etwa 2 mm. Die dünne Wandung gewährleistet die ausreichende Elastizität des Fersenkeils 4 mit seiner Wandung 10.

Die einheitliche Ausbildung des Zehenteils 8 und der Sohle 9 ermöglicht die kosmetische Ausgestaltung des Zehenteils 8 mit einzelnen Zehen 12. Eine derartige kosmetische Ausgestaltung war bei den durch die US-A-3 098 239 bekannten künstlichen Füßen nicht möglich, da die Sohle unverkennbar aus einem anderen Material als die Oberseite gebildet war, so daß die kosmetische Ausgestaltung des Zehenteils 8 nicht sinnvoll war.

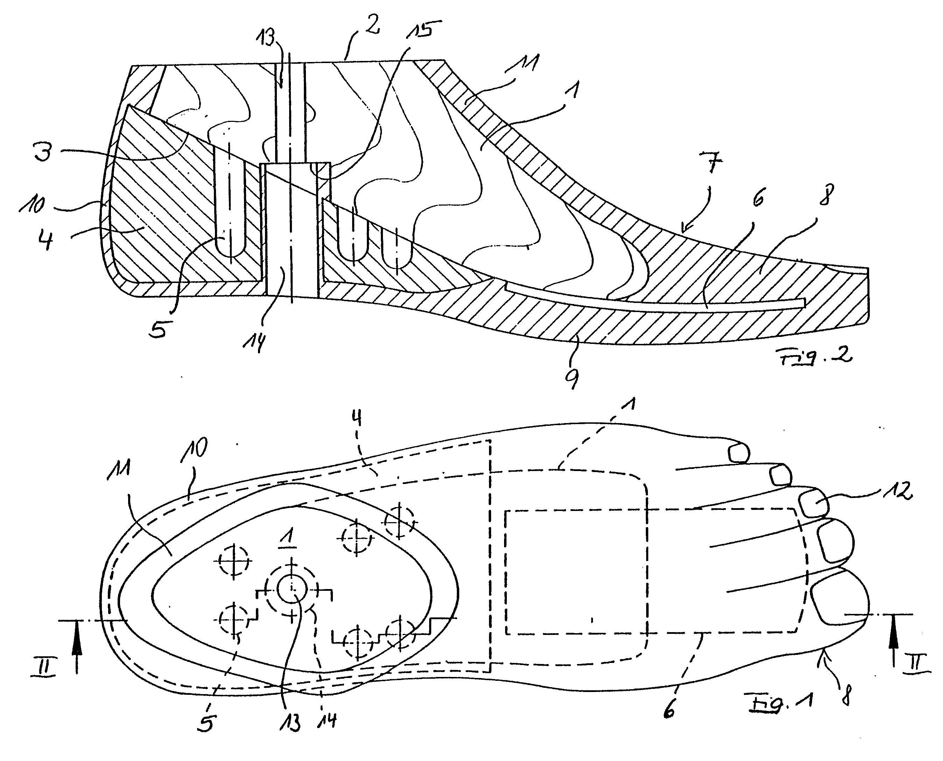

Figur 1 läßt die Verteilung der Sackbohrungen 5 erkennen, die zweckmäßigerweise so erfolgt, daß auf beiden Seiten der Längsachse des Fußes eine gleiche Anzahl von Sackbohrungen 5 vorhanden ist. Die Verteilung der Sackbohrungen in Längsrichtung auf jeder Seite erfolgt nach Belastungsgesichtspunkten beim Abrollen des Fußes entsprechend der Gewichtsverteilung.

Zur Befestigung des künstlichen Fußes an einem (nicht dargestellten) Beinteil weist der Fuß eine vertikale Bohrung 134 auf, die sich als Durchgangsbohrung von der Beinanschlußfläche 2 durch den Kern 1, den Fersenkeil 4 und die die untere Schutzwandung 10 für den Fersenkeil bildende Sohle 9 erstreckt. Die Bohrung 13 weist nach unten hin einen Teil 14 mit einem größeren Innendurchmesser auf. Somit kann eine Schraube mit dem Schraubenkopf nach unten von unten in die Bohrung 13 eingeführt werden, wobei der Schraubenkopf an dem durch den Durchmessersprung entstandenen Flansch 15 anliegt. Dieser Flansch 15 ist bei der Montage der Schraube vorzugsweise mit einer Dichtscheibe abgedeckt. Die Innenwandung des erweiterten Teils 14 der Bohrung 13 ist mit dem hydrolysefesten Kunststoff 7 ausgekleidet, der sich also nahtlos von der Sohle 9 in die Innenwandung des Bohrungsteils 14 bis zum Flansch 15 fortsetzt.

Nach Ansetzen des künstlichen Fußes an das Beinteil ist das aus Kern 1, Fersenkeil 4 und Textilband 6 bestehende Innere des Fußes hermetisch ummantelt, so daß weder Feuchtigkeit noch schädliche Agenzien in das Innere des Fußes gelangen können.

Durch die sehr weiche Ausgestaltung des Fersenkeils 4 und die Verwendung eines mittelharten hydrolysefesten Kunststoff 7 für die dünnwandige Ummantelung 10 des Fersenkeils 4 ergeben sich Laufeigenschaften, die den Laufeigenschaften der bisherigen künstlichen Füße nicht nachstehen. Der bisher für die Erhaltung der Laufeigenschaften in Kauf genommene Nachteil der mangelnden Beständigkeit des Fersenkeils und der Sohle der bekannten Füße kann somit durch den erfindungsgemäßen Fuß vermieden werden.

Es ist möglich, einen oder mehrere der Kanäle 5 für die genaue Zentrierung des Fersenkeils 4 an der Unterseite 3 des Kerns 1 auszunutzen. Gegebenenfalls kann hierzu der Kern 1 mit entsprechenden Sackbohrungen ausgestattet sein. In diese können beispielsweise leichte Zentrierstücke eingesetzt werden.

Die Kanäle 5 in dem Fersenkeil 4 bieten den weiteren Vorteil, daß die gesamte Anordnung des Fußes leichter wird. Der erfindungsgemäße Fuß kann daher sehr leicht erstellt werden. Unter diesem Aspekt ist auch die Verwendung eines Holzkerns 1 sinnvoll, weil dieser aus sehr leichtem Holz bestehen kann, das leichter als hierfür geeignete Kunststoffe ist.

**Patentansprüche**

1. Künstlicher Fuß mit einem sich in den Spann- und Knöchelbereich erstreckenden Kern (1) aus inkompressiblem Material, dessen Unterseite (3) schräg von vorn nach hinten ansteigend verläuft, mit einer den Kern (1) mit Ausnahme einer Beinanschlußfläche (2) umhüllenden, die Fußform bildenden Schicht aus einem Polyurethanschaum, in die im Fersenbereich unterhalb des Kerns (1) ein Fersenkeil (4) eingesetzt ist, wobei unterhalb der Beinanschlußfläche (2) eine vertikale Bohrung durch den Fuß hindurchläuft, deren Durchmesser nach unten im Bereich des Kerns (1) stufenförmig vergrößert ist, die zur Aufnahme einer Verschraubung dient und in dem Bohrungsteil (14) mit dem größeren Durchmesser mit dem Polyurethanschaum ausgekleidet ist, dadurch gekennzeichnet, daß der Fersenkeil (4) aus einem Vulkollanschaum mit einer spezifischen Masse von etwa 3 g/cm³ gebildet, an die schräg verlaufende Unterseite (3) des Kerns (1) angesetzt und seitlich sowie an seiner Unterseite von einer etwa 2 mm starken Wand (10) des Polyurethanschaums mit einer spezifischen Masse von etwa 6 g/cm³ umgeben ist.

2. Künstlicher Fuß nach Anspruch 1, dadurch gekennzeichnet, daß der durch den Durchmessersprung der Bohrung (13) gebildete Flansch (15) mit einer Dichtscheibe abgedeckt ist.

3. Künstlicher Fuß nach Anspruch 1 oder 2, dadurch gekennzeichnet, daß die Weichheit des Fersenkeils (4) durch einen Hohlraum (5) erhöht ist.

4. Künstlicher Fuß nach Anspruch 3, dadurch gekennzeichnet, daß der Hohlraum durch mehrere vertikale Kanäle (5) gebildet ist.

5. Künstlicher Fuß nach Anspruch 4, dadurch gekennzeichnet, daß die vertikalen Kanäle als Sackbohrungen (5) ausgebildet sind, deren offenes Ende zum Kern (1) zeigt, so daß der Kern (1) die Sackbohrungen (5) vollständig verschließt.

6. Künstlicher Fuß nach Anspruch 4 oder 5, dadurch gekennzeichnet, daß beidseitig der Längsachse des Fußes eine gleiche Anzahl von Kanälen (5) vorgesehen ist.

**Claims**

1. Artificial foot comprising a core (1) extended in an ankle area and an instep area and formed of incompressible material, the bottom surface (3) of said core (1) sloping upwardly from the front side to the rear side, a layer of a polyurethane foam surrounding the core (1) with exception of a leg connection surface (2) and forming the foot shape, in which layer a heel wedge (4) is inserted below the core (1) in the heel region, the foot being provided with a vertical bore extending through the foot below the leg connection surface (2), the diameter of the bore being steplike enlarged downwardly in the region of the core (1) and the bore serving for housing a screw means and being provided with a coating within that part (14) of the bore having the larger diameter, characterized in that the heel wedge (4) is formed of a vulkollan foam having a specific weight of about 3 g/cm³, is fixed to the sloping bottom surface (3) of the core (1) and es surrounded at its sides and its bottom surface by a wall (10) of a polyurethane foam having a specific weight of about 6 g/cm³ and being about 2 mm thick.

2. Artificial foot according to claim 1, characterized in that the flange (15) being formed by the diameter step of the bore (13) is covered by a sealing washer.

3. Artificial foot according to claim 1 or 2, characterized in that the grade of softness of the heel wedge (4) is enlarged by a hollow (5).

4. Artificial foot according to claim 3, characterized in that the hollow is formed by a number of vertical channels (5).

5. Artificial foot according to claim 4, characterized in that the vertical channels are formed as pockets (5) the open end of which is directed to the core (1) so that the core (1) completely closes the pockets (5).

6. Artificial foot according to claim 4 or 5, characterized in that on both sides of the longitudinal axis of the foot an identical number of channels (5) are provided.

**Revendications**

1. Pied artificiel comportant une âme centrale (1), en matériau incompressible, qui s'étend dans la région du cou-de-pied et dans la région de la cheville et dont la face inférieure (3) se développe en remontant obliquement de l'avant vers l'arrière, une couche d'une mousse de polyuréthane qui donne la forme du pied, qui entoure l'âme centrale (1) à l'exception d'une surface de raccordement à la jambe (2) et dans laquelle est placée, dans la région du talon, en dessous de l'âme centrale (1), une cale formant talon (4), un perçage vertical traversant le pied en dessous de la surface de raccordement à la jambe (2), ce perçage, dont le diamètre s'agrandit vers le bas suivant un décrochement dans la région de l'âme centrale (1), servant à recevoir un organe de vissage et étant revêtu de mousse de polyuréthane dans la partie du perçage (14) possédant le plus grand diamètre, caractérisé en ce que la cale formant talon (4) est constituée par une mousse de Vulkollan ayant une masse spécifique d'environ 3 g/cm³, est appliquée contre la face inférieure (3), se développant obliquement, de l'âme centrale (1), et est entourée sur ses côtés, ainsi que sur sa face inférieure, par une paroi (10), d'épaisseur environ 2 mm, de mousse de polyuréthane ayant une masse spécifique d'environ 6 g/cm³.

2. Pied artificiel selon la revendication 1, caractérisé en ce que le collet (15) formé par la variation brutale du diamètre du perçage (13) est recouvert d'une bague d'étanchéité.

3. Pied artificiel selon la revendication 1 ou 2, caractérisé en ce que la souplesse de la cale formant talon (4) est accrue par un espace creux (5).

4. Pied artificiel selon la revendication 3, caractérisé en ce que l'espace creux est constitué de plusieurs canaux verticaux (5).

5. Pied artificiel selon la revendication 4, caractérisé en ce que les canaux verticaux (5) sont constitués de perçages borgnes dont l'extrémité ouverte fait face à l'âme centrale (1), en sorte que l'âme centrale (1) obture complètement les perçages borgnes.

6. Pied artificiel selon une des revendications 4 ou 5, caractérisé en ce que, des deux côtés de l'axe longitudinal du pied, un nombre identique de canaux (5) est prévu.

Fig. 2

Fig. 1